(19) **Europäisches Patentamt / European Patent Office / Office européen des brevets**

(11) **EP 1 988 157 A1**

(12) **EUROPEAN PATENT APPLICATION**
published in accordance with Art. 158(3) EPC

(43) Date of publication:
**05.11.2008 Bulletin 2008/45**

(51) Int Cl.:
*C12N 5/00* (2006.01)     *C12N 5/10* (2006.01)
*C12N 15/09* (2006.01)

(21) Application number: **07714070.5**

(22) Date of filing: **13.02.2007**

(86) International application number:
**PCT/JP2007/052488**

(87) International publication number:
**WO 2007/094301 (23.08.2007 Gazette 2007/34)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **14.02.2006   JP 2006036953**

(71) Applicants:
- **Senju Pharmaceutical Co., Ltd.**
  **Osaka-shi, Osaka 541-0046 (JP)**
- **Yamashita, Hidetoshi**
  **Yamagata-shi, Yamagata 9902331 (JP)**

(72) Inventors:
- **YAMASHITA, Hidetoshi**
  **Yamagata-shi, Yamagata 990-2331 (JP)**

- **YAMAMOTO, Teiko**
  **Yamagata-shi, Yamagata 990-9585 (JP)**
- **NISHITSUKA, Kouichi**
  **Yamagata-shi, Yamagata 990-9585 (JP)**
- **KASHIWAGI, Yoshiko**
  **Yamagata-shi, Yamagata 990-9585 (JP)**
- **TOJO, Naoki**
  **Yamagata-shi, Yamagata 990-9585 (JP)**

(74) Representative: **Duckworth, Timothy John**
**J.A. Kemp & Co.,**
**14 South Square,**
**Gray's Inn**
**London WC1R 5JJ (GB)**

(54) **VITREOUS CELL LINE**

(57)    The present invention provides a vitreous cell line capable of expressing an exogenous immortalizing gene, and a production method thereof. Since the vitreous cell line of the present invention can produce a sufficient number of cells and has constant and continuous proliferative capacity, it can be advantageously utilized for the elucidation of the pathogenesis of retinal vitreous diseases, and the development of a drug for the prophylaxis and/or treatment of retinal vitreous diseases. Moreover, the cell line is not only highly useful for the biochemical·physiological studies of the vitreous body, and further for the study of cell differentiation mechanisms, but also possibly usable as a biological material of an artificial vitreous body.

EP 1 988 157 A1

## Description

## Technical Field

The present invention relates to a vitreous cell line, a production method thereof and the like.

## Background Art

The vitreous body is a gel-like tissue that fills the vitreous cavity surrounded by retina, ciliary body and crystalline lens. It forms an optic media and is also involved in the maintenance of the eyeball internal pressure. The vitreous body occupies about 4/5 of the eyeball volume, and 99% thereof is water. The gel structure of the vitreous body is a double mesh structure of collagen fiber and ball-like hyaluronic acid (HA), which affords the powerful water-containing ability. Abnormality in the vitreous body causes various retinal vitreous diseases (e.g., proliferative retinal vitreous disease etc.) and decreases the eyesight. It is important to analyze in detail the function and structure of the vitreous body for the development of a therapeutic drug for retinal vitreous diseases. The present inventors have reported that the production of hyaluronic acid is involved in the pathology of proliferative retinal vitreous diseases, based on the analyses of the proliferative tissues of the diseases (Japanese Journal of Ophthalmology, vol. 7, pp. 557-564, 2003: non-patent reference 1).

Vitreous cells are present within the vitreous body and produce a wide variety of vitreous constituent components such as HA and the like. Hence, it is important for the elucidation of the pathology of retinal vitreous diseases and advancement of the development of a therapeutic drug for the diseases to culture vitreous cells in vitro and analyze their functions.

However, the proliferative capacity of vitreous cells is considerably low to the extent that long-term culture is substantially impossible or, even if they could be cultured, the proliferation thereof is extremely difficult. In addition, vitreous cells obtained from a neonate can be cultured rather easily but the proliferative capacity thereof is limited, and gigantism and denaturation of the cells may occur in a long-term passage. As such, the development of a vitreous cell line permitting long-term passage while maintaining the inherent functions of the vitreous cell is desired.

In the meantime, a human crystalline epithelial cell line was produced by introducing a large T antigen gene of simian virus 40 (SV40) known to be an immortalizing gene into a human crystalline epithelial cell [JP-A-10-52272: patent reference 1, Andley U.P. et al., Invest. Ophthalmol. Vis. Sci., 35: 3094-3102 (1994): non-patent reference 2]. However, there has been no report on the establishment of the vitreous cell line.

patent reference 1: JP-A-10-52272
non-patent reference 1: Japanese Journal of Ophthalmology, 7: 557-564 (2003)
non-patent reference 2: Invest. Ophthalmol. Vis. Sci., 35: 3094-3102 (1994)

## Disclosure of the Invention

## Problems to be Solved by the Invention

It is therefore an object of the present invention to provide a vitreous cell line useful for the physiological·biochemical studies of the vitreous body and as a research tool for the development of a drug for the prophylaxis or treatment of retinal vitreous diseases such as a proliferative retinal vitreous disease and the like, which can be transplanted into living organisms for regeneration of the vitreous body.

## Means of Solving the Problems

The present inventors have succeeded in conferring in vitro infinite proliferative capacity on vitreous cells and establishing a clonal swine vitreous cell line that can be passaged semipermanently, by transfecting swine vitreous cells with a plasmid expression vector functionally incorporating an immortalizing gene, which resulted in the completion of the present invention.

Accordingly, the present invention relates to the following.

[1] A vitreous cell line capable of expressing an exogenous immortalizing gene.
[2] The cell line of [1], wherein the exogenous immortalizing gene encodes SV40 large T antigen.
[3] The cell line of [1], which is derived from swine.
[4] The cell line of [1], which has the following properties (1) - (3):

(1) no decrease in the proliferation rate;
(2) production capability of GFAP and S100; and
(3) promoted hyaluronic acid production when stimulated with TGF-$\beta$1 and PDGF-BB.

[5] The cell line of [4], further having the following property:

(4) increased expression of HAS2 when stimulated with PDGF-BB.

[6] The cell line of [4], further having the following property:

(5) increased expression of VEGF when stimulated with IL-1$\alpha$.

[7] The cell line of any of [4] to [6], wherein the above-mentioned properties are maintained for not less than 20 passages.
[8] The cell line of [1], which is established without infection with a live virus.

[9] The cell line of [1], which is established by transfecting an expression vector functionally harboring an exogenous immortalizing gene into vitreous cells and passaging the cells in a medium.

[10] A production method of a vitreous cell line, comprising transfecting an expression vector functionally harboring an exogenous immortalizing gene into vitreous cells and passaging the cells in a medium.

[11] The production method of [10], wherein the exogenous immortalizing gene encodes SV40 large T antigen.

**Effect of the Invention**

Since the vitreous cell line of the present invention can produce a sufficient number of cells and has constant and continuous proliferative capacity, it can be advantageously utilized for the elucidation of the pathogenesis of retinal vitreous diseases, and the development of a drug for the prophylaxis and/or treatment of retinal vitreous diseases. Moreover, the cell line is not only highly useful for the biochemical·physiological studies of the vitreous body, and further for the study of cell differentiation mechanisms, but also possibly usable as a biological material of an artificial vitreous body.

**Brief Description of the Drawings**

Fig. 1 is a photograph showing the morphology of a vitreous cell line.
Fig. 2 schematically shows examination protocol.
Fig. 3 shows the effect of PDGF-BB+TGF-$\beta$1 stimulation on HAS2 expression by a vitreous cell line.
Fig. 4 shows the effect of PDGF-BB+TGF-$\beta$1 stimulation on hyaluronic acid production by a vitreous cell line.
Fig. 5 shows the effect of forced expression of Smad2, 3 or 4 on HAS2 expression by stimulation with PDGF-BB and/or TGF-$\beta$1.
Fig. 6 schematically shows examination protocol.
Fig. 7 shows the effect of IL-1$\alpha$ stimulation on VEGF mRNA expression by a vitreous cell line.
Fig. 8 shows the effect of IL-1$\alpha$ stimulation on VEGF protein expression by a vitreous cell line, wherein the vertical axis shows relative values of swine VEGF concentration calculated based on a human VEGF standard product.
Fig. 9 shows the effect of IL-1$\beta$, TGF$\beta$, PDGF-AA, PDGF-BB or TNF$\alpha$ stimulation on VEGF mRNA expression by a vitreous cell line.

**Best Mode for Carrying out the Invention**

The vitreous cell line of the present invention is a cell group containing an exogenous immortalizing gene, which has acquired infinite proliferative capacity in vitro as a result of the expression of the immortalizing gene.

The vitreous cell line of the present invention is derived from a mammal. Examples of the mammal include rodents such as mouse, rat, hamster, guinea pig and the like, experiment animals such as rabbit and the like, artiodactyls such as swine, bovine, goat, sheep and the like, perissodactyls such as horse and the like, pets such as dog, cat and the like, primates such as human, monkey, Macaca mulatta, marmoset, orangutan, chimpanzee and the like; and the like. The vitreous cell line of the present invention is preferably derived from swine.

Here, the "immortalizing gene" refers to a gene that immortalizes cells and confers infinite proliferative capacity on the cells. Examples thereof include oncogenes such as c-myc, ras and the like, adenovirus E1A, SV40 derived large T antigen gene (SV40 large T antigen gene), large T antigen gene of polyoma virus, E6 and E7 genes of papilloma virus, and the like. In the present invention, the "exogenous immortalizing gene" means an immortalizing gene newly introduced extracellularly, which is not inherently possessed by the vitreous cell to be the origin of the vitreous cell line of the present invention. Therefore, the exogenous immortalizing gene includes, in addition to an immortalizing gene derived from a source other than the mammal from which the cell line of the present invention derives, for example, even an oncogene of the mammal from which the cell line of the present invention derives, which has been modified into a form that permits expression in a target cell (i.e., free of suppression of expression that normal endogenous oncogenes are under). As the exogenous immortalizing gene of the present invention, a virus-derived immortalizing gene, more preferably SV40 large T antigen gene, is preferably used.

The vitreous cell line of the present invention can be preferably established by transfecting vitreous cells separated from a tissue with an expression vector functionally harboring the above-mentioned exogenous immortalizing gene, namely, harboring the gene in a form allowing expression in a target cell, and passaging the cells in a suitable medium.

The vitreous cell can be isolated as fibroblast-like cells adhered onto a culture vessel by, for example, separating a vitreous tissue from an isolated eye tissue, cutting it small (about 2 mm square) with scissors etc., suspending the obtained vitreous fragment in a suitable liquid medium, for example, Eagle MEM medium, Dulbecco modified Eagle MEM medium, Ham-F12 medium, Katsuta medium DM-160 and the like containing 10 - 20% of fetal bovine serum or calf serum, culturing the fragments in a $CO_2$ incubator for about 2 days. Isolated vitreous cells are subjected to transfection.

The expression vector to be used in the present invention is exemplified by plasmid vectors and virus vectors. As the expression vector, a plasmid vector is preferable, since a cell line can be established without infection with a live virus in an immortalizing gene introduction process, and the possibility of virus particle production in the cell does not exist. When a virus genome is used as a vector, it is more preferable to delete or mutate the gene partially so that at least complete virus particles will not be produced in a cell harboring the vector.

As a construction method of an expression vector functionally harboring an exogenous immortalizing gene when, for example, a SV40 Large T antigen gene is used as an exogenous immortalizing gene, the following method can be mentioned. A DNA fragment containing an immortalizing gene present in the early region of SV40 genomic DNA (i.e., large T antigen gene) is cleaved out with a suitable restriction enzyme, and inserted in a plasmid vector containing a promoter capable of expressing the gene in a vitreous cell. Examples of the promoter include SV40 early promoter, Rous sarcoma virus (RSV) promoter, MuMLV LTR and the like. When SV40 initial promoter is used, a DNA fragment containing both the promoter and a large T antigen coding region may be cleaved out from the SV40 genomic DNA.

Examples of the plasmid vector include pBR322, pGEM and the like. Moreover, a transcription termination signal, a specific sequence that enhances the expression of an exogenous immortalizing gene and the like may be provided at a suitable site of the vector. The thus-constructed exogenous immortalizing gene expression vector is synthesized in a large amount in a suitable host, such as Escherichia coli, yeast, Bacillus subtilis etc., recovered and purified by a conventional method, and introduced into a vitreous cell by a conventional gene transfer method, such as calcium phosphate coprecipitation method, microinjection method, polyethylene glycol method, electroporation method and the like.

Examples of the virus vector include retroviruses such as moloney murine leukemia virus, lentivirus and the like, adenovirus, herpesvirus, adeno-associated virus, parvovirus, semliki forest virus, vaccinia virus, Hemagglutinating Virus of Japan and the like. Transgene by a retrovirus is preferable since the gene is introduced by incorporation into a chromosome. In addition, lentivirus can infect both dividing and non-dividing cells to introduce a gene. An exogenous immortalizing gene is introduced into a vitreous cell by culturing the vitreous cell in a culture medium containing virus particles. In this case, the introduction efficiency can be increased by using RetroNectin, fibronectin, polybrene and the like as a gene transfer reagent.

The cells after transfection are cultured in a liquid medium such as Eagle MEM medium, Dulbecco modified Eagle MEM medium, Ham-F12 medium, Katsuta medium DM-160 and the like. As the conditions of culture temperature, medium pH, $CO_2$ concentration and the like, general conditions conventionally used for culturing animal cells can be appropriately employed. The medium is exchanged with a fresh medium every 2 - 3 days, and when the cell proliferation becomes saturated, the cells are passaged. Even after termination of cell proliferation, the culture is continued and the group of cells that has started to grow again is divided as a monoclonal cell group. The clones can be separated as follows. A filter paper having a small diameter, which has been soaked with trypsin and EDTA solution, is placed still on the objective clonal cell group, and the cells are detached from a culture vessel and attached to the filter paper. The clonal cell group attached to the small filter paper piece is placed in a different culture vessel together with the filter paper. The clones can also be separated by a method of picking up the colony, a limiting dilution method or a method using a cell sorter. Since respective cells of a clonal cell line show uniform properties, they can be useful model cells for physiological·biochemical studies of the vitreous body, elucidation of pathogenesis of retinal vitreous diseases, development of a prophylactic or therapeutic drug for retinal vitreous diseases and the like, which have conventionally been performed using heterogeneous vitreous cells.

The vitreous cell line obtained as mentioned above can be passaged by a general animal cell cultivation technique. As an index of proliferative capacity of cell, a cell population doubling level (PDL) calculated by the following formula can be used.

$$PDL = \log_{10}(N_i/N_0)/\log_{10} 2$$

$N_i$ = number of cells at completion of the ith passage culture
$N_0$ = number of cells at start of the first passage culture

The PDL of the cell line of the present invention generally increases generally by about 1.1 - 2.0, preferably 1.4 - 1.5, per one passage.

The cell line of the present invention can have the following properties (1) - (3):

(1) no decrease in the proliferation rate;
(2) production capability of GFAP and S100; and
(3) promoted hyaluronic acid production when stimulated with TGF-β1 and/or PDGF-BB.

Here, production of GFAP and S100 is an index of the physiological characteristics as a vitreous cell possessed by the cell line of the present invention. GFAP (Glial Fibrillary Acidic Protein) is an intermediate filament having a molecular weight of about 50 kDa. S100 is an acidic protein having a molecular weight of about 10 kDa, which has two calcium binding motifs of loop-helix-loop structure called EF hand. Promoted hyaluronic acid production by stimulation with TGF-β1 and/or PDGF-BB is also an index of the physiological characteristics as a vitreous cell possessed by the cell line of the present invention. Since involvement of hyaluronic acid production in the pathology of proliferative retinal vitreous diseases and the like has been reported (Japanese Journal of Ophthalmology, vol. 7, p. 557-564, 2003), the cell line of the present invention particularly becomes a powerful tool for studying the involvement, in these diseases, of hyaluronic acid production from a vitreous cell by cytokine stimulation.

The cell line of the present invention may further have the following property (4), in addition to the above-mentioned properties:

(4) increased expression of HAS2 when stimulated with PDGF-BB.

The "expression of gene" means expression of mRNA encoding the gene or a gene product (protein) thereof. HAS2 (Hyaluronan synthase 2) is a synthesis enzyme of hyaluronic acid. Increased expression of HAS2 by stimulation with PDGF-BB is also an index of the physiological characteristics as a vitreous cell possessed by the cell line of the present invention.

The cell line of the present invention may further have the following property (5), in addition to the above-mentioned properties:

(5) increased expression of VEGF by stimulation with IL-1α.

For example, when the cell line of the present invention is stimulated with IL-1α, the VEGF mRNA level as measured by RT-PCR analysis and the like becomes 2-fold or more in 12 hours, and 2.5-fold or more in 48 hours, as compared to the mRNA level of non-stimulation control. Increased expression of VEGF by stimulation with IL-1α is also an index of the physiological characteristics as a vitreous cell possessed by the cell line of the present invention.

Moreover, the VEGF expression of the cell line of the present invention does not show a significant change by IL-1β, TGFβ, PDGF-AA, PDGF-BB or TNFα stimulation. For example, even when the cell line of the present invention is stimulated with these cytokines, the VEGF mRNA level in 48 hours as measured by RT-PCR analysis and the like is within the range of 0.5- to 1.5-fold of the mRNA level of non-stimulation control. Therefore, the cell line of the present invention is particularly useful for the analysis of the mechanism of VEGF expression by IL-1α stimulation.

Generally, the cell line of the present invention is substantially free of gigantism·denaturation of cells. Being "substantially free of gigantism·denaturation of cells" means that partially elongated cells may be found by a microscopic observation of the cells, but the majority (for example, about 95% or more) thereof shows the inherent morphological characteristics of a vitreous cell. When the cell line of the present invention is passaged for a long period (for example, about 40 passages or more), the cell morphology tends to be elongated. Such elongated cell line is also encompassed in the present invention.

In the vitreous cell line of the present invention, the above-mentioned properties are stably maintained for 20 passages or more, preferably 35 passages or more.

The cell line of the present invention can be a useful model cell for the physiological·biochemical studies of the vitreous body, elucidation of pathogenesis of retinal vitreous diseases and development of a prophylactic or therapeutic drug for the diseases, as well as safety tests of pharmaceutical products for ophthalmic diseases, and the like. In addition, since stable supply of the cell is possible, it can be a useful material for transplantation aiming at vitreous regeneration and an artificial vitreous body. Moreover, since the cell line of the present invention has production capability of GFAP and S100, which are vitreous specific proteins, and hyaluronic acid production capability, such substances can be produced in large amounts by large culture of the cell line. Since VEGF expression is promoted by stimulating the cell line of the present invention with IL-1α, a compound capable of preventing or treating the symptoms such as proliferation of vascular endothelium, promotion of vascular permeability, proliferation of neovessels and the like, which are mediated by VEGF released from vitreous cells, can be screened for by selecting a compound that suppresses induction of the expression.

The present invention is explained in more detail in the following by referring to Examples, which are not to be construed as limitative.

[Example 1]

(Isolation of swine vitreous tissue and preparation of swine vitreous cell)

The vitreous body was removed from the eye tissue of a 2-year-old swine, and cut into 2 mm square fragments with scissors. The obtained vitreous body fragments were suspended in DMEM (GIBCO) (containing 10% FBS, 100 U/ml penicillin and 100 μg/ml streptomycin), and cultured on a 10 cm culture dish at 37°C, 5% $CO_2$. Two days later, vitreous cells were obtained as fibroblast-like cells adhered onto the dish.

(Introduction of immortalizing gene SV40 large T antigen and isolation of cell line)

A vector harboring the sequence of SV40 large T antigen (RIKEN Tag-LTR-pUC18 sense #4) was introduced into the vitreous cell obtained above, by the calcium phosphate method (BD Calphos Mammalian Transfection Kit).
At 80% confluent, 0.25% trypsin/EDTA (4 ml) was added to the culture dish and the cells were incubated at 37°C, 5% $CO_2$ for 5 min. The cells were detached, and trypsin was deactivated by adding the same amount of the above-mentioned DMEM. Then, the mixture was centrifuged at 380×g for 2 min and the cells were collected. The cells were sown on a culture dish at 1:10 dilution, and further cultured at 37°C, 5% $CO_2$.

After culturing for 4 days, the cells forming colonies were selected, detached with trypsin, and cultured in a 12 well plate for each colony. At about 80% confluent, the cells were passaged into a 60 mm culture dish and when the cells became about 80% confluent, they were cryopreserved (5% DMSO/10% FBS/DMEM).

(Passage of cells)

The isolated colonies were continuously cultured until the 40th passage under similar conditions. The cells could be continuously passaged at a constant ratio throughout the culture, and therefore, the cell proliferation rate did not decrease. PDL was calculated every 5 passages. The PDL of the obtained cell line increased by about 1.4 - 1.5 for every passage.

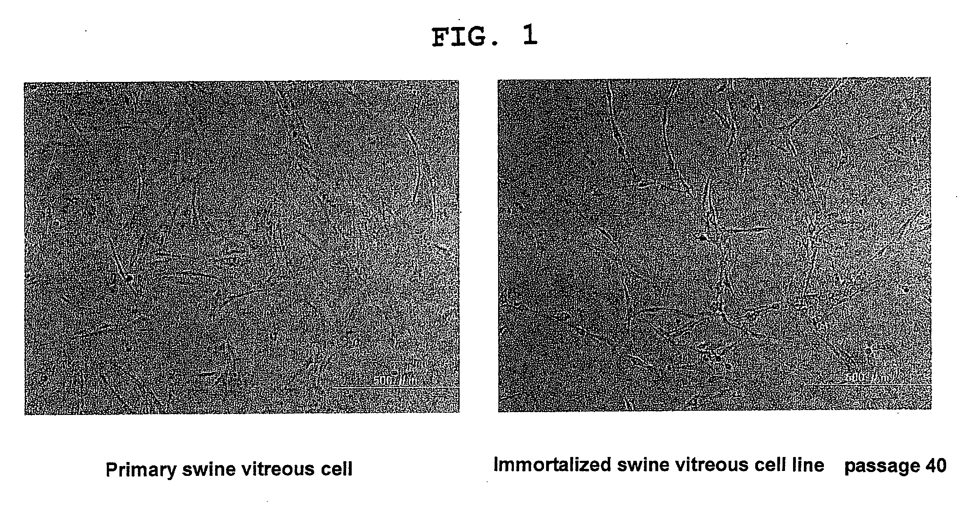

Cell morphology was observed under a microscope. The appearance of the cells was photographed with a cooling CCD camera DP-70 (manufactured by OLYMPUS) connected to a DMIRBE microscope (manufactured by Leica). The photographing conditions were as follows: objective 5-fold/ocular 10-fold/quantity of light 9.0 V. The cells consisted of a single layer with a fibroblast-like morphology (Fig. 1). Although a part of the cells after 40 passages tended to show an elongated cell morphology, gigantism and denaturation of the cells were hardly observed.

(Confirmation of SV40 Large T antigen expression)

Total RNA was isolated from the cells, cDNA was synthesized, and the mRNA expression of immortalizing gene SV40 large T antigen was confirmed by the RT-PCR method.

(Analysis of vitreous cell line phenotype)

The vitreous cell line expressed GFAP and S100 proteins. This shows that the obtained vitreous cell line maintains physiological characteristics as a vitreous cell.

Next, expression of hyaluronic acid synthase (HAS)2 and the effect of PDGF-BB and/or TGF-β1 on the hyaluronic acid production in the established vitreous cell line were examined. The expression was observed by RT-PCR.

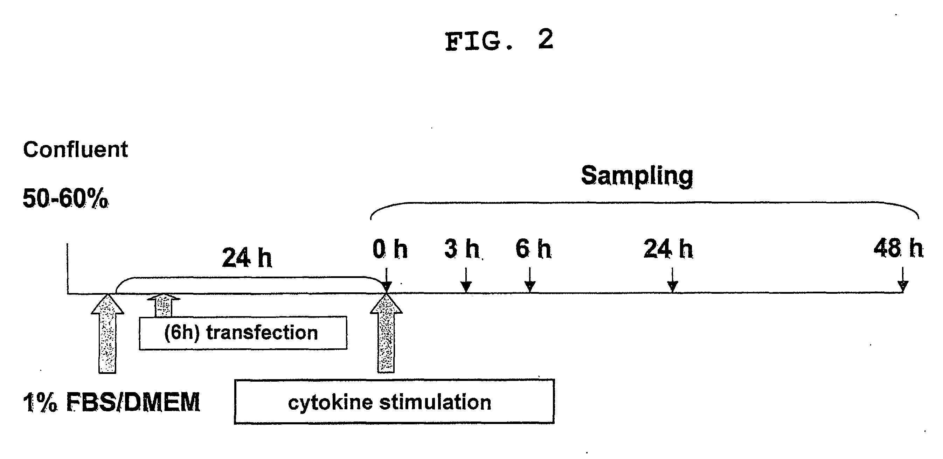

To be specific, when the vitreous cell line was 50 - 60% confluent, the medium of the cells was exchanged with DMEM containing 1% bovine serum and, after culturing for 24 hr, the cells were stimulated with TGF-β1 (10 ng/ml) and PDGF-BB (10 ng/ml). In a part of the examination, Smad2, 3 or 4 was transfected into the cell line after 6 hr from the medium exchange. The cells and culture supernatant were recovered at 3, 6, 24 and 48 hr after the cytokine stimulation (Fig. 2). Total RNA was extracted from the cells, cDNA was synthesized, and RT-PCR was performed using primers specific to HAS2. Using Roche FastStart High Fidelity PCR System, PCR was performed on Gene Amp PCR System 9700 (Applied Biosystems). The PCR conditions were as follows:

95°C 2 min. → (95°C 30 seconds → X°C 30 seconds → 72°C 30°C) ×Y cycle → 72°C 7 min
X=50, Y=35···HAS2
X=60, Y=25···beta-Actin

Furthermore, the hyaluronic acid concentration in the culture supernatant was measured using a hyaluronic acid measurement kit (Seikagaku Corporation).

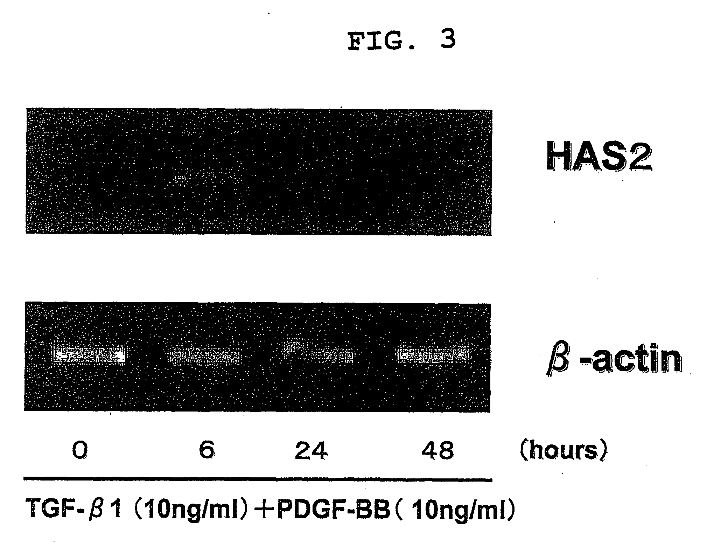

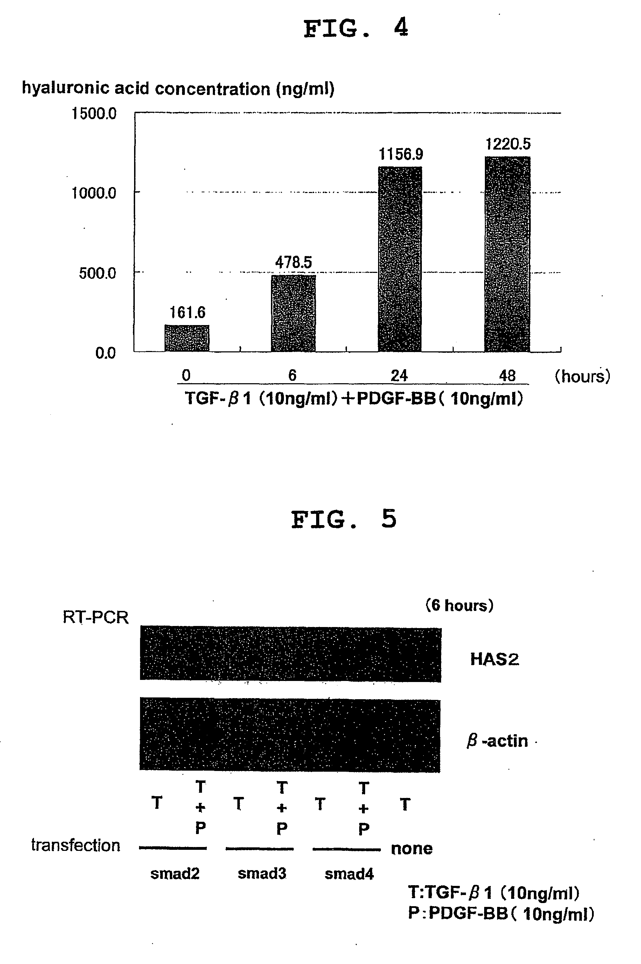

As a result, when the vitreous cell line was stimulated with TGF-β1 (10 ng/ml) and PDGF-BB (10 ng/ml), the mRNA expression level of HAS2 increased in 6 hours after the stimulation (Fig. 3). Since the stimulation increased the concentration of HA in the culture supernatant with time, it was suggested that the hyaluronic acid synthesis in the vitreous cell line was promoted by the stimulation with TGF-β1 and PDGF-BB (Fig. 4). This shows that the obtained vitreous cell line maintains physiological characteristics as a vitreous cell.

The forced expression of Smad2, 3 or 4 resulted in the expression of HAS2 by the stimulation with TGF-β1 (Fig. 5). This suggests that the action of TGF-β1 is mediated by the pathway of Smad2, 3 or 4. A synergistic effect of TGF-β1 and PDGF-BB was not observed.

[Example 2]

Changes of VEGF expression due to cytokine stimulation in the vitreous cell line established in Example 1 were examined. The expression of VEGF at an mRNA level was examined by RT-PCR, and the expression at a protein level was examined by ELISA.

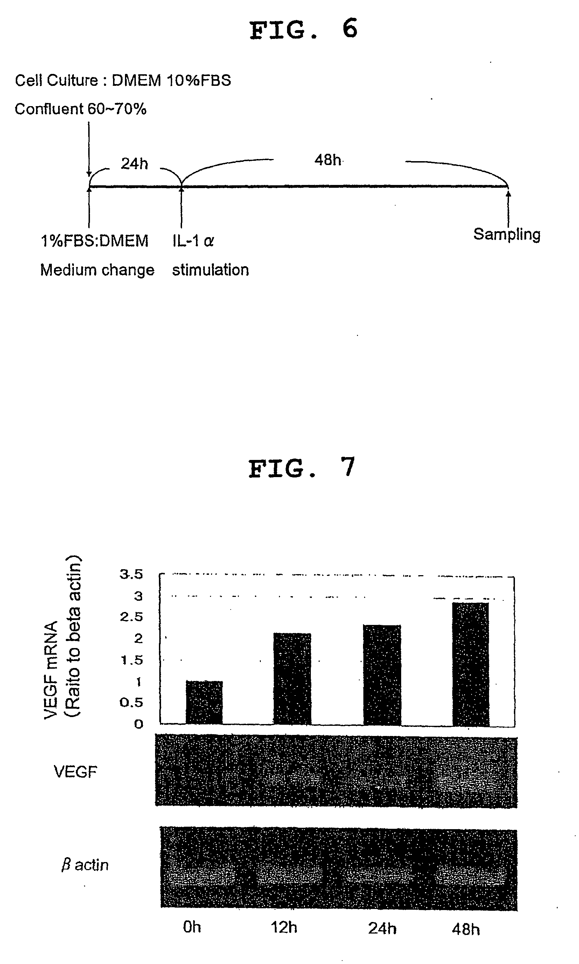

To be specific, when the vitreous cell line reached 50 - 60% confluent, the medium for the cells was exchanged with DMEM containing 1% bovine serum. After culturing for 24 hours, the cells were stimulated with cytokine (IL-1α, IL-1β, TNFα, PDGF-AA, PDGF-BB or TGFβ) (10 ng/ml) for 48 hours (Fig. 6). Total RNA was extracted from the stimulated cells, cDNA was synthesized, and PCR was conducted using VEGF primers. The medium of the stimulated cells was used for ELISA. Since an appropriate antibody to swine VEGF is not available, VEGF protein concentration was measured by ELISA using an antibody to human VEGF, and the result was calculated as a reference value led from a human VEGF standard product. PCR was conducted using TOYOBO KOD plus kit and Gene PCR System 9700 (Applied Biosystem). The conditions of PCR were as follows:

95°C 120 seconds;
(95°C 15 seconds, 62°C 30 seconds, 68°C 30 seconds) x30 cycles; 68°C 90 seconds.

ELISA was entrusted to Mitsubishi BCL.

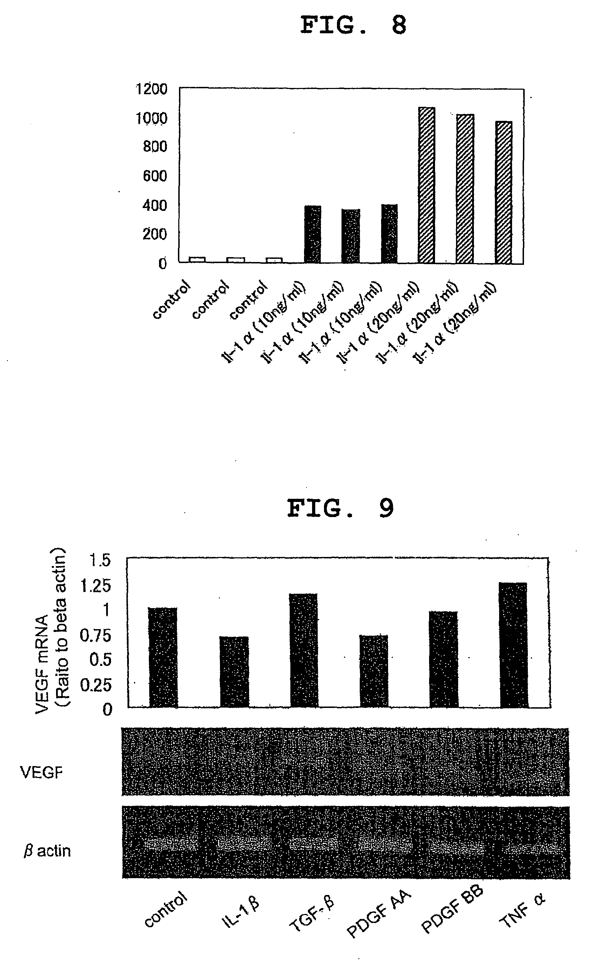

As a result, when the vitreous cell line was stimulated with IL-1α, the VEGF mRNA expression level increased with time (Fig. 7). Since the concentration of VEGF protein in the culture supernatant also increased with time due to the stimulation, it was suggested that the production of VEGF protein in the vitreous cell line was promoted by the stimulation with IL-1α (Fig. 8).
In contrast, even when the vitreous cell line was stimulated with other cytokines (IL-1β, IL-6, TNFα, PDGF-AA, PDGF-BB or TGFβ), a significant change in the VEGF mRNA expression level was not observed (Fig. 9).
This suggests that the vitreous cell line obtained in Example 1 is particularly useful for the analysis of the mechanism of VEGF expression by stimulation with IL-1α. Suppression of VEGF expression by IL-1α stimulation is considered to lead to the suppression of the proliferation of vascular endothelium, suppression of the promotion of vascular permeability and suppression of the proliferation of neovessels.

## Industrial Applicability

Since the vitreous cell line of the present invention can produce a sufficient number of cells and has constant and continuous proliferative capacity, it can be advantageously utilized for the elucidation of the pathogenesis of retinal vitreous diseases, and the development of a drug for the prophylaxis and/or treatment of retinal vitreous diseases. Moreover, the cell line is not only highly useful for the biochemical·physiological studies of the vitreous body, and further for the study of cell differentiation mechanisms, but also possibly usable as a biological material of an artificial vitreous body.
This application is based on a patent application No. 2006-036953 filed in Japan (filing date: February 14, 2006), the contents of which are incorporated in full herein by this reference.

## Claims

1. A vitreous cell line capable of expressing an exogenous immortalizing gene.

2. The cell line of claim 1, wherein the exogenous immortalizing gene encodes SV40 large T antigen.

3. The cell line of claim 1, which is derived from swine.

4. The cell line of claim 1, which has the following properties (1) - (3):

    (1) no decrease in the proliferation rate;
    (2) production capability of GFAP and S100; and
    (3) promoted hyaluronic acid production when stimulated with TGF-β1 and PDGF-BB.

5. The cell line of claim 4, further having the following property:

    (4) increased expression of HAS2 when stimulated with PDGF-BB.

6. The cell line of claim 4, further having the following property:

    (5) increased expression of VEGF when stimulated with IL-1α.

7. The cell line of any of claims 4 to 6, wherein said properties are maintained for not less than 20 passages.

8. The cell line of claim 1, which is established without infection with a live virus.

9. The cell line of claim 1, which is established by transfecting an expression vector functionally harboring an exogenous immortalizing gene into vitreous cells and passaging the cells in a medium.

10. A production method of a vitreous cell line, comprising transfecting an expression vector functionally harboring an exogenous immortalizing gene into vitreous cells and passaging the cells in a medium.

11. The production method of claim 10, wherein the exogenous immortalizing gene encodes SV40 large T antigen.

# FIG. 1

Primary swine vitreous cell

Immortalized swine vitreous cell line    passage 40

EP 1 988 157 A1

FIG. 2

EP 1 988 157 A1

# FIG. 3

HAS2

$\beta$ -actin

0     6    24    48  (hours)

TGF-$\beta$ 1 (10ng/ml)＋PDGF-BB( 10ng/ml)

# FIG. 4

hyaluronic acid concentration (ng/ml)

TGF-$\beta$1 (10ng/ml)＋PDGF-BB( 10ng/ml)

# FIG. 5

RT-PCR (6 hours)

HAS2

$\beta$-actin

transfection

smad2  smad3  smad4

T:TGF-$\beta$1 (10ng/ml)
P:PDGF-BB( 10ng/ml)

# FIG. 6

Cell Culture : DMEM 10%FBS

Confluent 60~70%

24h    48h

1%FBS:DMEM    IL-1 α

Medium change    stimulation

Sampling

# FIG. 7

VEGF mRNA
(Raito to beta actin)

3.5
3
2.5
2
1.5
1
0.5
0

VEGF

β actin

0h        12h        24h        48h

# FIG. 8

# FIG. 9

## INTERNATIONAL SEARCH REPORT

| | International application No. |
|---|---|
| | PCT/JP2007/052488 |

A.  CLASSIFICATION OF SUBJECT MATTER
*C12N5/00*(2006.01)i, *C12N5/10*(2006.01)i, *C12N15/09*(2006.01)n

According to International Patent Classification (IPC) or to both national classification and IPC

B.  FIELDS SEARCHED

Minimum documentation searched (classification system followed by classification symbols)
C12N5/00, C12N5/10, C12N15/09

Documentation searched other than minimum documentation to the extent that such documents are included in the fields searched

Electronic data base consulted during the international search (name of data base and, where practicable, search terms used)
BIOSIS/MEDLINE/WPIDS/CAplus(STN), JMEDPlus/JST7580/JSTPlus(JDream2)

C.  DOCUMENTS CONSIDERED TO BE RELEVANT

| Category* | Citation of document, with indication, where appropriate, of the relevant passages | Relevant to claim No. |
|---|---|---|
| X/Y | EVANS I. M., et al., Establishment of adenovirus E1A immortalized hyalocytes and eye endothelial cells for the study of blood vessel regression., Invest. Ophthalmol. Vis. Sci., 1997, vol. 38, no. 4 PART1-2, p. S790 | 1,9,10/2-8, 11 |
| X/Y | JP 2002-112764 A (Kabushiki Kaisha Tohoku Techno Arch), 16 April, 2002 (16.04.02), Par. Nos. [0004] to [0007]; example 1 (Family: none) | 1,2/3-11 |
| X/Y | YANAI N., et al., A tubule cell line established from transgenic mice harboring temperature-sensitive simian virus 40 large T-antigen gene., Jpn. J. Cancer Res., 1991, vol. 82, no. 12, p. 1344-1348 | 1,2/3-11 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 05 March, 2007 (05.03.07) | 13 March, 2007 (13.03.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

## INTERNATIONAL SEARCH REPORT

| International application No. |
| --- |
| PCT/JP2007/052488 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 11-508142 A  (Neurotech S.A.),<br>21 July, 1999 (21.07.99),<br>Claim 4<br>& WO 1997/040139 A       & EP 0833895 A | 1-11 |
| Y | JP 10-052272 A  (Senju Pharmaceutical Co.,<br>Ltd.),<br>24 February, 1998 (24.02.98),<br>Claims 1 to 6<br>& EP 0798375 A | 1-11 |
| Y | ANDLEY U. P., et al., Propagation and<br>immortalization of human lens epithelial cells<br>in culture., Invest. Ophthalmol. Vis. Sci.,<br>1994, vol. 35, no. 7, p. 3094-30102 | 1-11 |
| P,X | Hirokazu NISHIZUKA et al., "Garasutai Saibo no<br>Bunshi Byotai ni Okeru Yakuwari", Journal of<br>Japanese Ophthalmological Society, 2006.03.15,<br>vol. 110, p. 198(1-P-A-09) | 1-11 |
| T | Yasuaki HATA, "Garasutai Saibo no Seiriteki·<br>Byoteki Igi", The Journal of the Eye, 2005,<br>vol. 22, no. 4, p. 489-491 | 1-11 |
| T | Toshinori IDE, "Jikken Igaku Bioscience 13 Hito<br>Saibo no Roka to Fushika", Kabushiki Kaisha<br>Yodosha, 1994, p.56-63 | 1-11 |
| A | JP 2005-524633 A  (University of South Florida),<br>18 August, 2005 (18.08.05),<br>& WO 2003/065999 A       & EP 1575524 A | 1-11 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10052272 A

- JP 2006036953 A

**Non-patent literature cited in the description**

- *Japanese Journal of Ophthalmology,* 2003, vol. 7, 557-564
- **ANDLEY U.P et al.** *Invest. Ophthalmol. Vis. Sci.,* 1994, vol. 35, 3094-3102

- *Invest. Ophthalmol. Vis. Sci.,* 1994, vol. 35, 3094-3102